(19) Europäisches Patentamt
European Patent Office
Office européen des brevets

(11) Veröffentlichungsnummer: **0 086 946**
**B1**

# (12) EUROPÄISCHE PATENTSCHRIFT

(45) Veröffentlichungstag der Patentschrift: **26.02.86**

(51) Int. Cl.⁴: **C 07 D 413/10**, C 07 D 405/10, C 07 D 403/10, C 08 K 5/34, C 08 K 5/35, D 06 L 3/12

(21) Anmeldenummer: **83100330.6**

(22) Anmeldetag: **15.01.83**

(54) **Azolylstyrylverbindungen.**

(30) Priorität: **30.01.82 DE 3203058**

(43) Veröffentlichungstag der Anmeldung: **31.08.83 Patentblatt 83/35**

(45) Bekanntmachung des Hinweises auf die Patenterteilung. **26.02.86 Patentblatt 86/9**

(84) Benannte Vertragsstaaten: **CH DE FR GB LI**

(56) Entgegenhaltungen:
**EP - A - 0 002 042**
**EP - A - 0 006 171**
**EP - A - 0 009 095**
**EP - A - 0 020 298**
**EP - A - 0 022 491**
**DE - A - 1 955 065**

**JOURNAL OF THE AMERICAN CHEMICAL SOCIETY, Band 80, 1958, Easton, W.G. FINNEGAN et al. "An improved synthesis of 5-substituted tetrazoles", Seiten 3908-3911**

(73) Patentinhaber: **BAYER AG, Konzernverwaltung RP Patentabteilung, D-5090 Leverkusen 1 Bayerwerk (DE)**

(72) Erfinder: **Eckstein, Udo, Dr., Wolfskaul 8, D-5000 Köln 80 (DE)**

**EP 0 086 946 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung sind Verbindungen der Formel

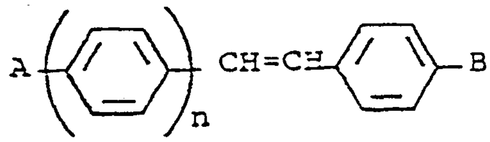

(I)

oder deren Isomerengemiche,worin

A einen gegebenenfalls durch R, R', O4, CN, OR, COR, $SO_2R$, NHCOR, $CONH_2$, $NHSO_2R$, OCOR, COOR, COOH, NHR' oder $SO_3H$ substituierten Benzoxazolyl-2- oder Benz-s-triazol-2-Rest oder einen Naphtho-s-triazolyl-2-Rest, worm R' für Alkyl und R fü Alkenyl , Aralkyl, Cycloalkyl oder Aryl stehen,

B eninen Rest der Formeln

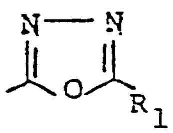

oder

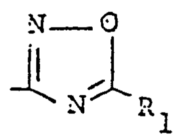

n 1 oder 2

$R_1$ Wasserstoff, Chlor, Amino, $C_1$-$C_4$-Alkylamino, Phenylamino oder einen Rest der Formel

$$-(OCH_2CH_2)_m-OW$$

W Wasserstoff, gegebenenfalls durch Chlor oder Cyano substituiertes $C_1$ -$C_6$-Alkyl, Benzyl, Cyclohexyl oder Phenyl und

m eine ganze Zahl von 0 bis 7 bedeuten,

sowie deren Verwendung als optische Aufheller oder Laserfarbstoffe.

Von den strukturell nächstvergleichbaren Ayrylverbindungen gemäß DE-A 19 55 065 unterscheiden sich diese neuen Verbindungen durch eine Äthergruppierung in den Oxdiazolylresten und ein Stilbengerüst im "mittleren" Teil des Moleküls.

Bevorzugte Verbindungen sind solche der Formel I, worin

A für den Rest der Formel

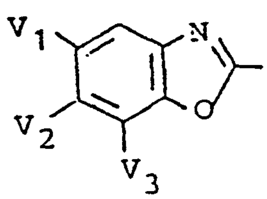

und

B für den Rest der Formel

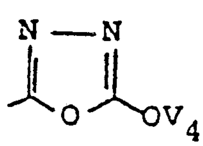

stehen,

wobei

n 1 und

worin

$V_1$ Wasserstoff, $C_1$-$C_4$-Alkyl, Cyclohexyl, $C_1$ -$C_4$-Alkoxy, Chlor, Benzyl, Phenyl, $C_1$-$C_4$-Alkoxycarbonyl, $C_1$ -$C_4$-Alkylsulfonyl

$V_2$ Wasserstoff, $C_1$-$C_4$-Alkyl, Chlor oder $C_1$ -$C_4$-Alkoxy,

$V_3$ Wasserstoff, $C_1$-$C_4$-Alkyl oder Chlor und

$V_4$ gegebenenfalls durch $C_1$-$C_4$-Alkoxy, Hydroxy, Chlor oder Cyano substituiertes $C_1$-$C_6$-Alkyl, Benzyl, Cyclohexyl oder Phenyl bedeuten.

Ganz besonders bevorzugt stehen $V_4$ für einen unsubstituierten $C_1$-$C_4$-Alkylrest wie Methyl, Ethyl, n-Propyl, Isopropyl oder n-Butyl; $V^1$ für Wasserstoff, Methyl, Chlor; $V^2$ und $V^3$ für Wasserstoff.

Die Verbindungen der Formel I können nach verschiedenen, an sich bekannten Methoden hergestellt werden. Vorzugsweise kondensiert man

a) eine Phosphono-Verbindung der Formel

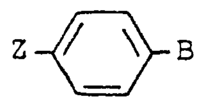

(II)

worin

Z für eine Gruppierung der Formeln

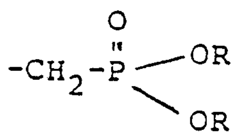
,
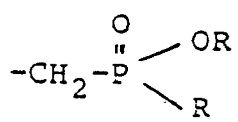
,
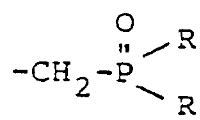

mit einem Aldehyd der Formel

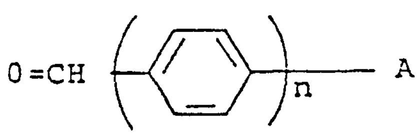

(III)

oder

b) eine Phosphono-Verbindung der Formel

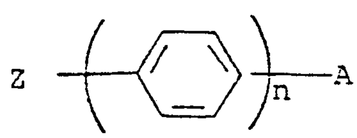

(IV)

mit einem Aldehyd der Formel

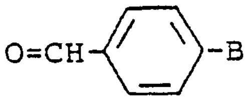

(V)

in organischen Lösungsmitteln in Gegenwart basischer Kondensationsmittel (vgl. z.B. DE-OS 2 525 684, 2 833 470 und 3 013 279). Bevorzugte Reste R sind $C_1$-$C_4$-Alkyl, Cyclohexyl oder Phenyl.

Als Lösungsmittel wählt man vorteilhafterweise indifferente, beispielsweise Kohlenwasserstoffe wie Toluol oder Xylol oder Alkohole wie Methanol, Ethanol, Isopropanol, Butanol, Glykol, Glykolether wie 2-Methoxyethanol, Hexanol, Cyclohexanol, Cyclooctanol, ferner Ether wie Diisopropylether, Dioxan, Tetrahydrofuran, weiterhin Formamide oder N-Methylpyrrolidon. Besonders geeignet sind dipolar-aprotische Lösungsmittel wie Dimethylformamid und Dimethylsulfoxid.

Als Kondensationsmittel kommen stark basische Verbindungen in Betracht wie Alkali- und Erdalkalimetallhydroxide, Alkali- und Erdalkaliamide und Alkaliund Erdalkalimetallalkoholate, beispielsweise Kaliumhydroxid, Natriumhydroxid, Kalium-tert.-Butylat, Natriumamid oder Natriummethylat, ferner die Alkaliverbindungen des Dimethylsulfoxids und Alkalihydride sowie gegebenenfalls Alkalimetalldispersionen.

Man arbeitet vorzugsweise im Temperaturbereich von 0 bis 100°C.

Die erfindungsgemäßen Verbindungen der Formel I können auch in der Weise hergestellt werden, daß man die entsprechenden Aldehydanile in einem dipolar aprotischen Lösungsmittel, wie Dimethylformamid in Gegenwart von basischen Kondensationsmitteln mit den entsprechenden Methylverbindungen umsetzt.

Während die Verbindungen III und IV allgemein bekannt sind (vgl. DE-OS 1 294 917, 2 148 014, 2 453 355, 2 709 924 und 2 926 234 sowie US-PS 3 351 591 und 4 12 044), sind die Verbindungen der Formeln II und V ($R_1$ Wasserstoff) bislang nicht in der Literatur beschrieben worden; man erhält diese Verbindungen in an sich bekannter Weise nach folgendem Reaktionsschema:

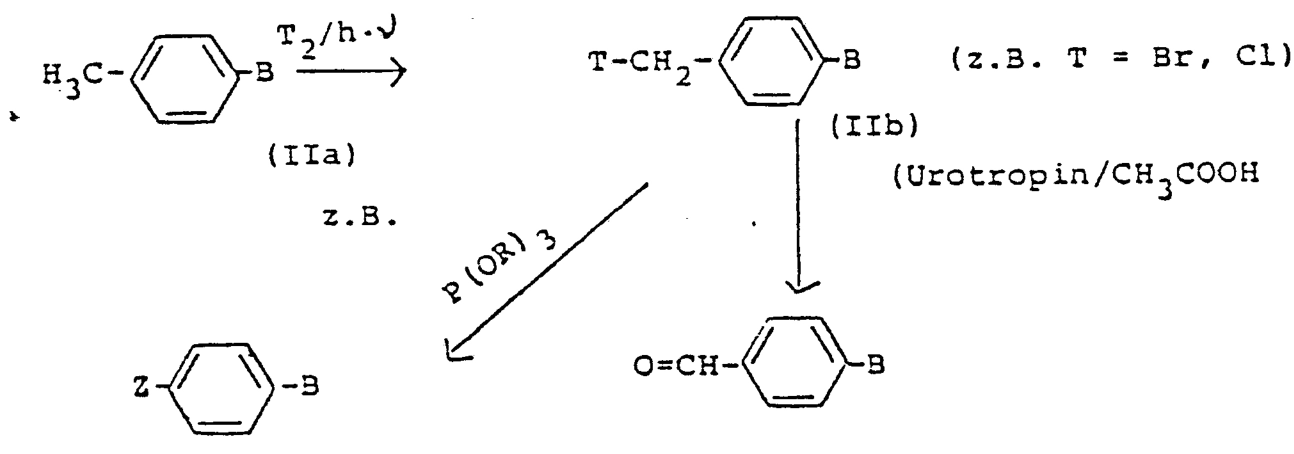


(II) (V)

Die Ausgangsverbindungen (IIa) (B = 1,3,4-Oxadiazol-rest) können nach verschiedenen literaturbekannten Verfahren hergestellt werden (vgl. z. B. Gazz. chim. ital. 91, 866 (1961) Tetrahedron Letters 42 (1964) 3119 und Ann. 686 (1965) 145).

Die Kondensation von Amidoxi-men mit Chlorameisensäureestern (vgl. Ber. 18, 2465 (1885) und Ber. 19, 1481 (1886) sowie Ber. 18 2456 (1885) und Ber. 19 1475 (1886)) und die anschließende Umsetzung mit entsprechenden Halogeniden in Gegenwart von Basen liefert die Edukte (IIa) bzw. auch direkt (II) (B = 1,2,4-Oxadiazolinon-2-rest).

Die Edukte (IIa) (B = 1,2,4-Oxadiazol-rest) schließlich erhält man in an sich bekannter Weise aus den unsubstituierten Oxadiazolinon-2-Verbindungen durch Chlorierung und Umsetzung mit Alkoholen bzw. Aminen (vgl. z. B. Bull. Soc. Chim. Belges 78(1969) 41 und Bull. Soc. Chim. Belges 78, 47 (1969).

Ein besonders vorteilhaftes Verfahren zur Herstellung von Verbindungen der Formel I ist dadurch gekennzeichnet, daß man Verbindungen der Formel

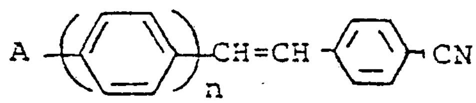

(VI)

mit Metallaziden in polaren organischen Lösungsmitteln zu Verbindungen der Formel

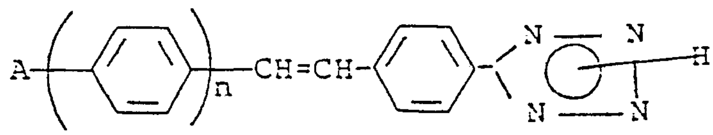

(VII)

umsetzt und dieses mit Verbindungen der Formel

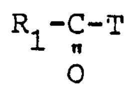

zu Verbindungen der Formel

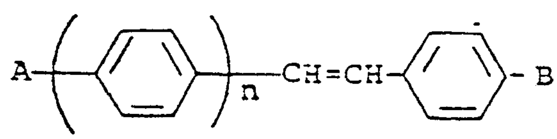

(VIII)

umsetzt.

Bei dieser Herstellungsmethode kann die Verbindung VIII auch, je nach Reaktionsführung in Form von Isomerengemischen anfallen die gewßnschten falls durch übliche Trennmethoden (Umkristallisieren, Chromatographie) getrennt werden.

Für die weiter unten angegebenen technischen Zwecke ist jedoch eine derartige Trennung nicht erforderlich.

Die Verbindungen der Formel VI sind nur zum Teil bekannt. Man erhält sie in üblicher Weise z.B. durch Umsetzung von Verbindungen der Formel

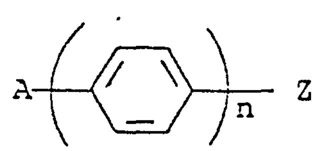

(IV)

mit p-Cyanobenzaldehyd (vgl. DE-OS 24 53 355, DE-AS 1 052 405, Jp-QS 49/85378).

Die Umsetzung der Tetrazol-Derivate VII mit den Verbindungen $R_1COT$ kann im Bereich von 50-15O°C, vorzugsweise bei 70-120°C durchgeführt werden.

Die Reaktionen werden in indifferenten Lösungsmitteln beispielsweise Ethern wie Dioxan, Tetrahydrofuran, Diisopropylether, ferner in Kohlenwasserstoffen wie Toluol, Xylol, Chlorbenzol und 1,2-Dichlorbenzol oder in

Formamiden wie Dimethylformamid, Dimethylacetamid oder Dimethylsulfoxid, vorzugsweise in Gegenwart von Säureakzeptoren, insbesondere tertiären organischen Basen wie Triethylamin, Dimethylanilin, Pyridin, Dimethylanilin oder Hexahydrodimethylanilin durchgeführt.

Die Reaktionstemperaturen für die erfindungsgemäße Umsetzung liegen zweckmäßig zwischen 100 ung 250° C, vorzugsweise zwischen 120 und 200° C.

Die Aufhellung des Fasermaterials mit der wäßrigen oder evtl. organischen Aufhellerflotte erfolgt nach dem für die jeweilige Faserart typischen Färbeverfahren.

An den Reaktionsprodukten der vorstehenden Verfahren können noch weitere, an sich bekannte Umwandlungen vorgenommen werden, wie Halogenierungen, funktionelle Abwandlungen von Carboxylgruppen, Einführung von Chlormethylgruppen oder Austausch von Halogenatomen gegen Cyanogruppen.

Die Verbindungen der Formel I zeigen im gelösten oder fein verteilten Zustand eine sehr starke blaue Fluoreszenz. Sie eignen sich einzeln oder als Mischungen zum Weißtönen der verschiedensten organischen Materialien.

Als aufzuhellende Substrate seien beispielsweise folgende Materialien genannt: Lacke, natürliche oder synthetische Fasern, wie z. B. solche aus natürlicher oder regenerierter Cellulose, Acetylcallulose, natürlichen und synthetischen Polyamiden, Polyestern, Polyolefinen, Polyvinylchlorid, Polyvinylidenchlorid, Polystyrol oder Polyacrylnitril sowie Folien, Filme, Bänder oder Formkörper aus solchen Materialien.

Die in Wasser unlöslichen erfindungsgemäßen Verbindungen können gelöst in organischen Lösungsmitteln zum Einsatz kommen oder in wäßriger Dispersion, vorteilhaft unter Zuhilfenahme eines Dispergierungsmittels, eingesetzt werden.

Die Menge der erfindungsgemäß zu verwendenden Verbindungen der allgemeinen Formel I, bezogen auf das optisch aufzuhellende Material, kann je nach Einsatzgebiet und gewünschtem Effekt in weiten Grenzen schwanken. Sie kann durch Versuche leicht ermittelt werden und liegt im allgemeinen zwischen etwa 0,01 und etwa 2 %.

Verbindungen der Formel I sind mit ihrer großen Quantenausbeute und hohen Lichtechtheiten außerdem weiterhin verwendbar für durchstimmbare Farbstofflöser im blauen Spektralbereich von 400-480 nm. Zu diesem Zweck werden sie in der Anordnung eingesetzt, wie sie in der DE-OS 1 910 784 bzw. GB-PS 1 255 399 beschrieben wird.

**Beispiel 1**

Eine Suspension aus 51,5 g (0,16 Mol) 4-Cyano-4'-(benz-oxazol-2"yl)-stilben, 11,4 g (0,175 Mol) Natriumazid und 7,4 g (0,175 Mol) Lithiumchlorid in 850 ml Dimethylformamid werden 25 Stunden bei 130° C verrührt. Es wird mit konzentrierter Salzsäure auf pH 3-4 gestellt und filtriert. Waschen mit Wasser und Ethanol liefert nach Trocknen im Vakuum bei 50° C 60,6 g (94,8 % d. Th.) der Verbindung der Formel

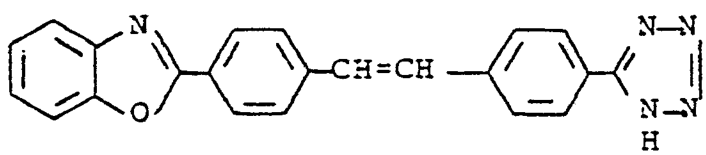

(1)

die aus Dimethylacetamid umkristallisiert werden kann. (Schmp.: 321'C Zers., τ max. 369 nm).

**Beispiel 2**

11 g (0 03 Mol) der Verbindung (1) aus Beispiel 1 und 7 1 g (0,07 Mol) Triethylamin werden in 100 ml Dimethylformamid 1 Std. bei 80° C verrührt. Unter die Oberfläche der Lösung werden dann 3,3 g (0,03 Mol) Chlorameisensäureethylester getropft. Die Mischung wird 2 Std. bei 80° C verrührt und erneut mit 3,3 g (0,03 Mol) Chlorameisensäureethylester behandelt. Nach 2 Std. bei ca. 90° C wird auf 10° C abgekühlt und vom nicht umgesetzten Ausgangsprodukt abfiltriert. Das Filtrat wird i. Vac. eingeengt, der Rückstand mit 50 ml Wasser behandelt und abfiltriert. Trocknen im Vakuum bei 50° C liefert 8,5 g (69,1 % d.Th.) hellgelbe Kristalle der Verbindung der Formel

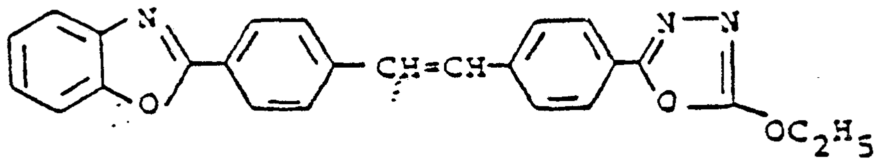

(2)

die aus Chlorbenzol umkristallisiert werden können. Schmp. 209-211°C, Absorption: λp +5a fert brillante Weißeffekte auf PES im Auszieh-HT- und Thermosol-Verfahren.

**Beispiel 3**

Analog Beispiel 2 ergibt die Umsetzung der Verbindung (3) mit Chlorameisensäuremethylester 9,2 g (68,8 % d.Th.) der Verbindung der Formel

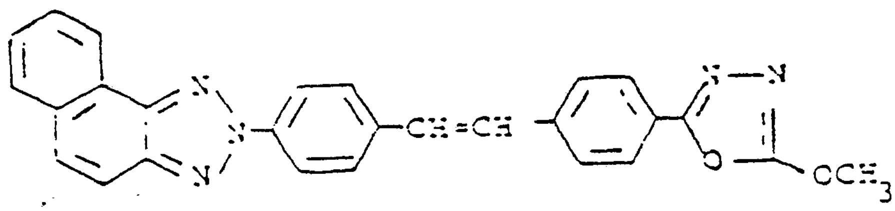

(3)

als gelbe Kristalle, die aus 1,2-Dichlorbenzol umkristallisiert werden können (Schmp. 229-32°C; Flureszenz in DMF: $\lambda_{max}$: 372 nm).

**Beispiel 4**

Zu einer Lösung von 22,7 g (0,08 Mol) 5-Ethoxy-2-(4'-brom-methylphenyl)-1,3,4-oxadiazol in 80 ml Toluol tropft man bei 100°C unter Stickstoff 16,6 g Triethylphosphit so zu, daß das entstehende Bromethan sofort abdestilliert. Man rührt 4 Stunden bei ca. 110°C und entfernt dann das Toluol und überschüssiges Triethylphosphit im Vakuum. Zum Rückstand werden 100 ml Dimethylformamid und 19 g (0,08 Mol) 2-(4'-Formyl-phenyl)-5-methyl-benzoxazol addiert. Bei 50°C werden zur Mischung 100 ml (0,1 Mol) einer 1 molaren Natriummethylat-Lösung getropft und 4 Stunden bei 60°C nachgerührt. Die Mischung wird auf Raumtemperatur abgekühlt, mit 6,6 g (0,11 Mol) Eisessig neutralisiert und mit 100 ml Ethanol versetzt. Abfiltrieren, Waschen mit Ethanol und Trocknen im Vakuum bei 50°C liefert 20,1 g (59,5 % d.Th.). Sehr sauberes Rohprodukt der Formel

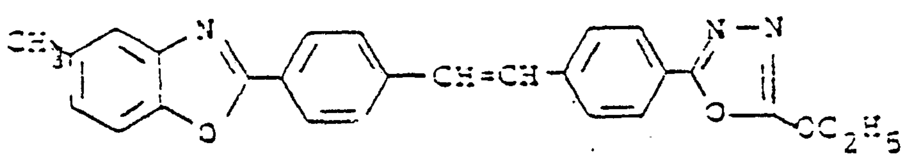

(4)

das aus Xylol umkristallisiert wird (Schmp.: 215°C, $\lambda_{max}$ : 366 nm). Die Verbindung liefert hervorragende Weißeffekte aus PES mit sehr guten Echtheiten.

2-(4'-Brom-methylphenyl)-5-ethoxy-1,3,4-oxadiazol wird in an sich bekannter Weise aus der entsprechenden Tolylverbindung durch Bromieren mit N-Bromsuccinimid in Tetrachlorkohlenstoff hergestellt.

In analoger Weise, wie in den Beispielen 2-4 beschrieben, werden auch die folgenden Verbindungen hergestellt:

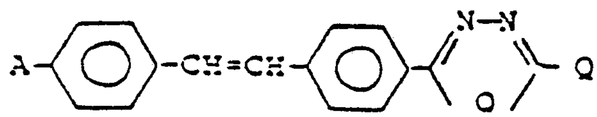

| Nr. | A | Q | Fluoreszenz in DMF |
|---|---|---|---|
| 5 | 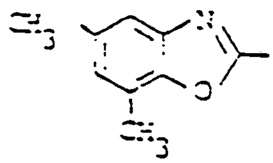 | $-OCH_3$ | blau |
| 6 | 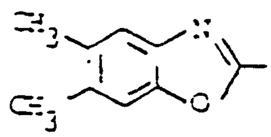 | $-OC_2H_5$ | neutr. blau |
| 7 | 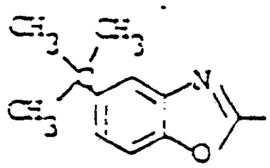 | $-OCH_2CH_2OCH_3$ | neutr. blau |
| 8 | 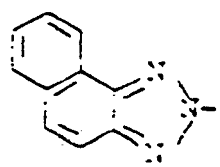 | $-OC_4H_9$ | blau |
| 9 | 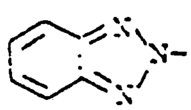 | $-OCH_2CH_2Cl$ | neutr. blau |

| Nr. | A | Q | Flureszenz in DMF |
|---|---|---|---|
| 10 | 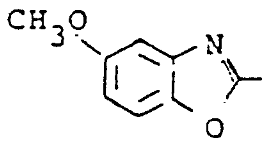 | $-OCH_3$ | intensiv blau |
| 11 | 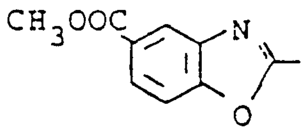 | 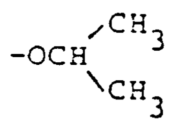 | violett blau |
| 12 | 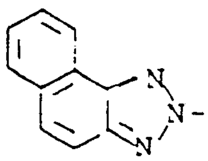 | $-OCH_2CH_2OC_2H_5$ | blau |
| 13 | 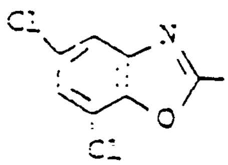 | $-NH_2$ | grünst. blau |

**Beispiel 14**

Zu einer Suspension von 13,7 g (0,05 Mol) 2-(4-Formyl-phenyl)-naphth/1,2-d/-1,2,3-triazol und 15,6 g (0,05 Mol) 3-(4-Diethoxyphosphonomethyl-phenyl)-1,2,4-oxadiazol-5-on in 150 ml wasserfreiem Dimethylformamid werden bei 50°C innerhalb von 30 Min. 20 g (0,11 Mol) 30 %ige Natriummethylat-Lösung getropft. Die Mischung wird 3 Std. bei 50-60°C verrührt und danach mit 20 g konz. Essigsäure versetzt. Nach dem Abkühlen wird abfiltriert und nacheinander mit je 50 ml 5 %iger Salzsäure, Wasser und Methanol gewaschen. Trocknen im Vak. bei 50°C liefert 13,6 g (63 % d. Theorie) hellgelbes Kristallpulver der Verbindung der Formel

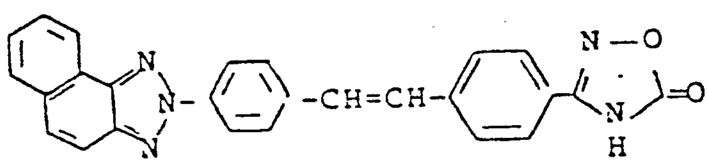

(14)

das aus Dimethylformamid umkristallisiert werden kann. Schmp: > 300°C, IR (KBr): 3430 $cm^{-1}$ 1760 $cm^{-1}$ , 1599 $cm^{1}$, UV-Absorption (DMF):$\lambda_{max}$ = 374 nm.

Die verwendete Diethoxy-phosphono-methylverbindung der Formel

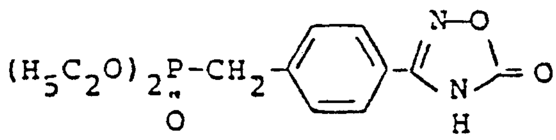

wird auf folgende Weise hergestellt:

28 g (0,4 Mol) Hydroxylaminhydrochlorid und 20 g (0,2 Mol) Natriumcarbonat werden in 300 ml dest. Wasser gelöst. Zur Lösung gibt man tropfenweise 27,8 g (0,11 Mol) 4-(Diethoxy-phosphonomethyl)-benzonitril und rührt 2 Std. bei 80°C. Nach Abkühlen auf Raumtemperatur wird 3 x mit je 100 ml Chloroform extrahiert und die vereinigten Chloroformphasen über wasserfreiem Natriumsulfat getrocknet. Das Lösungsmittel wird im Vac. entfernt und das erhaltene Rohöl in 100 ml wasserfreiem Xylol gelöst. Man fügt 17,8 g (0,11 Mol) Pyrokohlensäureethylester hinzu und steigert die Innentemperatur langsam, wobei zunächst Ethanol später Xylol abdestilliert (etwa 2-3 Std). Nach Beendigung der Reaktion wird das Lösungsmittel zur Hälfte durch Destillation entfernt und abgekühlt. Abfiltrieren und Trocknen im Vakuum bei 50°C liefert 26,2 g (76,3 % d. Theorie) farblose Kristalle vom Schmp. 196-201°C die aus Ethanol umkristallisiert werden können

| $C_{13}H_{17}N_2O_5$ P (312,3) | | C % | H % | N % |
|---|---|---|---|---|
| | Ber.: | 50,0 | 5,49 | 8,97 |
| | Gef.: | 49,9 | 5,4 | 8,6 |

**Beispiel 15**

11,2 g (0,05 Mol) 2-(4-Formyl-phenyl)-benzoxazol und 15,6 g (0,05 Mol) 3-(4-Diethoxy-phosphonomethylphenyl)-1,2,4-oxadiazolin-5-on werden in 100 ml wasserfreiem Dimethylformamid suspendiert und in der gleichen Weise wie in Beispiel 14 beschrieben, umgesetzt und isoliert.

Man erhält 15 2 g (79,7 % d. Theorie) hellgebe Kristalle der Verbindung der Formel

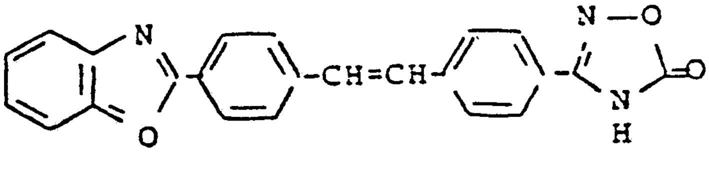

(15)

die aus Dimethylformamid umkristallisiert werden können. Schmp: <300°C; IR (KBr): 3425 $cm^{-1}$, 1760 $cm^{-1}$, 1605 $cm^{-1}$; UV-Absorption (in DMF) $\lambda_{max} = 362$ nm.

**Beispiel 16**

Analog Beispiel 14 erhält man aus 14,1 g (0,05 Mol) 2-(4-Formyl-phenyl)-5-carbmethoxy-benzoxazol und 15,6 g (0,05 Mol) 3-(4-Diethoxy-phosphonomethyl-phenyl)-1,2,4-oxa-diazolin-5-on 16,5 g (75,1 % d. Theorie) hellgelbe Kristalle der Verbindung der Formel

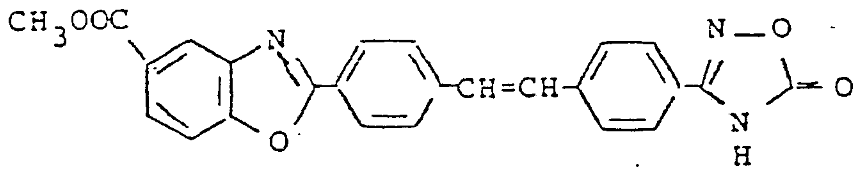

(16)

die aus Dimethylacetamid umkristallisiert werden kann. Schmp. < 300°C; IR (KBr) 3420 $cm^{-1}$ , 1765 $cm^{-1}$ , 1735 $cm^{-1}$, 1600 $cm^{-1}$ UV-Absorption (in DMF) $\lambda_{max}$ = 364 nm.

**Beispiel 17**

8,63 g (0,02 Mol) der Verbindung 14 werden in 100 ml wasserfreiem Dimethylformamid suspendiert und 4 g (0,022 Mol) 30 %ige Natriummethylat-Lösung hinzugefügt. Nach 1 Std. Rühren bei 50°C werden 3,74 g (0,022 Mol) 2-Jod-propan zugetropft und die Reaktionsmischung 4 Std. bei 80°C verrührt. Bei 50°C wird abfiltriert, das Filtrat bis zur Trockne eingeengt und mit 50 ml Wasser und 10 gkonz. Essigsäure behandelt. Abfiltrieren und Trocknen im Vakuum bei 50°C liefert 6,1 g (64,4 % d. Th.) gelbes Kristallpulver der Verbindung der Formel

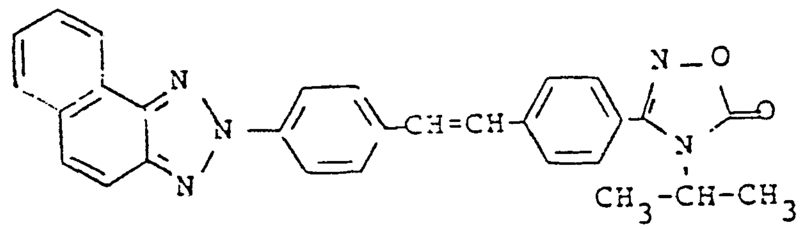

(17)

das aus Dimethylacetamid umkristallisiert werden kann (UV-Absorption (DMF):$\lambda_{max}$ = 372 nm; IR(KBr) 1775 $cm^{-1}$, 1605 $cm^{-1}$).

**Beispiel 18**

Zur Suspension von 11,9 g (0,05 Mol) 2-(4-Formyl-phenyl)-5-methyl-benzoxazol und 15,6 g (0,05 Mol) 3-(4-Diethoxy-phosphonomethyl-phenyl)1,2,4-oxadiazolin-5-on in 100 ml wasserfreiem Dimethylformamid tropft man bei 50°C innerhalb von 30 Min. 20 g (0,11 Mol) 30 %ige Natriummethylat-Lösung. Die Mischung wird bis zur vollständigen Umsetzung etwa 3 Std. bei 50-60°C gerührt. Nun gibt man 8,4 g (0,055 Mol) λ-Bromessigsäuremethylester zu und rührt 3 Std. bei 90°C. Nach dem Abkühlen auf Raumtemperatur fügt man 20 g konz. Essigsäure hinzu, filtriert den gelben Niederschlag ab, wäscht mit Wasser und Methanol und trocknet. im Vak. bei 50°C. Ausbeute 15,3 g (65,4 % d. Theorie) der Verbindung der Formel

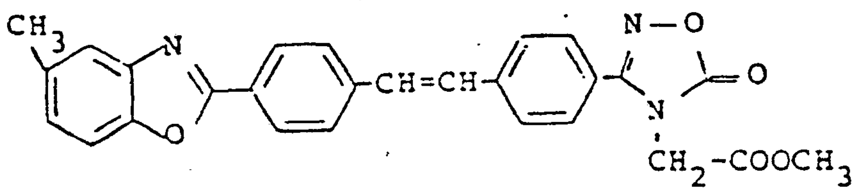

(18)

die aus Methylglykol umkristallisiert werden können. (UV-Absorption (DMF):$\lambda_{max}$ = 364 nm).
In analoger Weise werden auch die folgenden Verbindungen hergestellt:

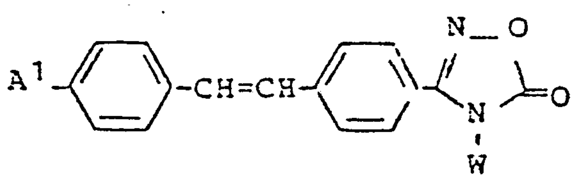

| Nr. | $A_1$ | W | Fluoreszenz in DMF |
|---|---|---|---|
| 19 | 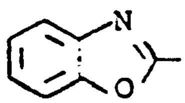 | $-CH_2-COOC_2H_5$ | rotst. Blau |
| 20 | 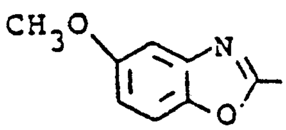 | H | rotviolett-blau |
| 21 | -"- | $CH_3$ | rotst. Blau |
| 22 | 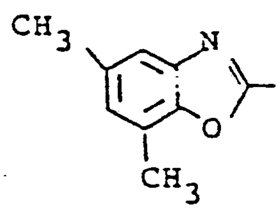 | $-C_4H_9$ | blau |
| 23 | 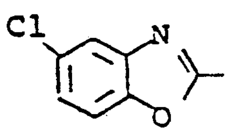 | $-CH_2-$ 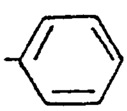 | sehr rotst. Blau |
| 24 | 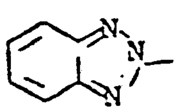 | $-CH_2-CH=CH_2$ | neutrales Blau |

| Nr. | $A_1$ | W | Fluoreszenz in DMF |
|---|---|---|---|
| 25 | 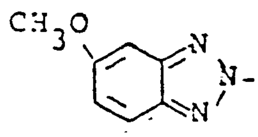 | H | wenig grünst. Blau |
| 26 | 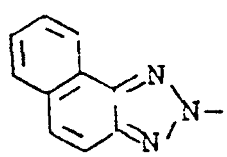 | $CH_2-COOC_2H_5$ | sehr starkes blau |

**Beispiel 27**

Zu einer Lösung von 22,7 g (0,08 Mol) 5-Ethoxy-3-(4-brommethyl-phenyl)-l,2,4-oxadiazol in 80 ml abs. Toluol tropft man bei 100°C unter Stickstoff 20 g Triethylphosphit so zu, daß das entstehende Bromethan sofort abdestilliert. Man rührt 5 Std. bei ca. 100°C und entfernt das Toluol und überschüige Triethylphopshit im Vakuum. Zum Rückstand werden 100 ml Dimethylformamid und 21,9 g (0,08 Mol) 2-(4-Formyl-phenyl)-naphth/1,2-d/-1,2,3-triazol addiert. Bei 50°C werden zur Mischung 100 ml (0,1 Mol) einer 1 molaren Natriumethylat-Lösung getropft und 4 Std. bei 50-60°C nachgerührt. Die Mischung wird auf Raumtemperatur abgekühlt, mit 6,6 g (0,11 Mol) Eisessig neutralisiert und abfiltriert. Waschen mit Ethanol und Trocknen im Vakuum bei 50°C liefert 23,0 g (62,5 % der Theorie) gelbe Kristalle der Verbindung der Formel

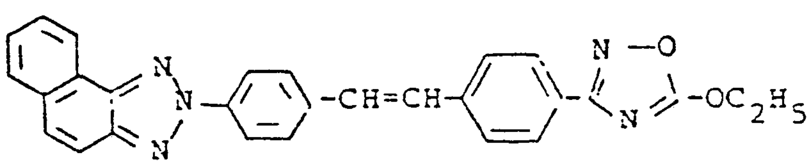 (27)

die aus Chlorbenzol umkristallisiert werden können (UV-Absorption (in DMF) $\lambda_{max} = 370$ nm). 5-Ethoxy-3-max (4-brommethyl-phenyl)-1,2,4-oxadiazol erhält man aus 3-(4-methyl-phenyl)1,2,4-oxadiazol (vgl. F. Eloy, A. Deryckere, A. van Overstraeten Bull. Soc. Chim. Belges 78 (1969) 47-54) in bekannter Weise durch Bromieren mit N-Bromsuccinimid in Tetrachlorkohlenstoff.

**Beispiel 28**

Analog beispiel 27 erhält man aus 11,2 g (0,05 Mol) 2-(4-Formyl-phenyl)-benzoxazol und 14,2 g (0,05 Mol) 5-Ethoxy-3-(4-brommethylphenyl)-1,2,4-oxadiazol, 15,5 g (75,6 % der Theorie) der Verbindung der Formel

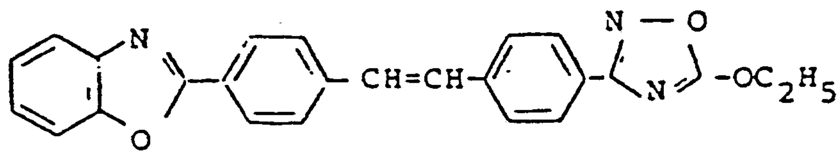

(28)

die aus Chlorbenzol umkristallisiert wird (UV-Absorption (in DMF)$\lambda_{max}$ = 360 nm).
In analoger Weise werden auch die folgenden Verbindungen hergestellt:

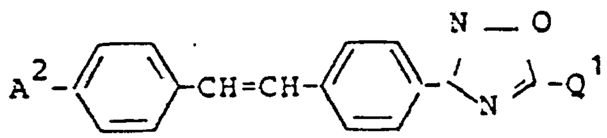

| Nr. | $A^2$ | $Q^1$ | Flureszenz in DMF |
|---|---|---|---|
| 29 | 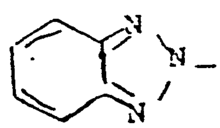 | $-OCH_3$ | stark blau |

| Nr. | $A^2$ | $Q^1$ | Fluoreszenz in DMF |
|---|---|---|---|
| 30 | 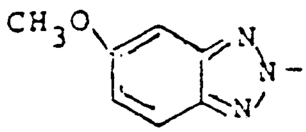 | $-OCH_3$ | etwas grünst. blau |
| 31 | 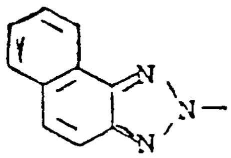 | $-OCH_2CH_2-OCH_3$ | stark blau violett |
| 32 | 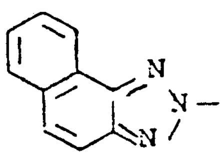 | $-OC_4H_9$-n | blau violett |
| 33 | 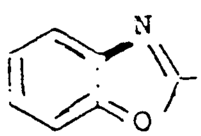 | $-OCH_2-$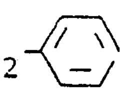 | rotst. blau |
| 34 | 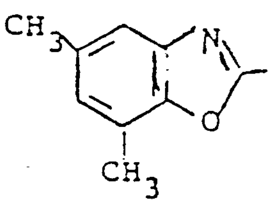 | $-OC_3H_7$-i | blau |
| 35 | 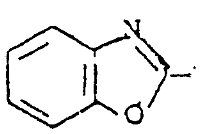 | -NH-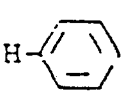 | grünst. blau |

**Beispiel 36**

14 5 g (0 03 Mol) Verbindung 27 werden in 100 ml Dimethylfornamid 3 Std. am Rückfluß erhitzt. Nach dem Abkühlen auf Raumtemperatur werden 50 ml Methanol hinzugefügt und das ausgefallene Produkt abfiltriert, mit Methanol gewaschen und bei 50°C im Vakuum getrocknet. Man erhält 11 g (75,9 % der Theorie) der Verbindung der Formel

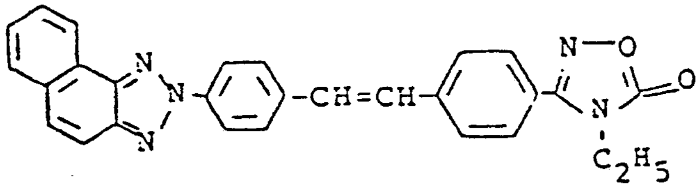

(36)

die aus Dimethylacetamid umkristallisiert wird (UV-Absorption (in DMF) :$\lambda_{max}$ = 373 nm).

**Patentansporüche**

1. Verbindungen der Formel

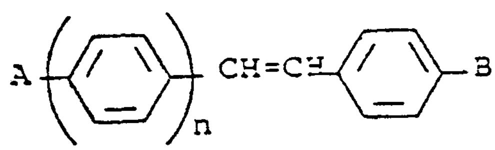

(I)

oder deren Isomerengemische, worin

A einen gegebenenfalls durch R, R', OH, CN, OR, COR, $SO_2$ R, NHCOR, $CONH_2$ $NHSO_2R$, OCOR, COOR, COOH, NHR' oder $SO_3H$ substituierten Ben-zoxazolyl-2- oder Benz-s-triazolyl-2-Rest, oder einen Naphto-s-triozolyl-2-Rest worin R' für Alkyl und R für Alkenyl, Arolkyl, Cycloalkyl oder Aryl stehen'

B einen Rest der Formeln >FIG32/35mm

n 1 oder 2, $R_1$ Wasserstoff, Chlor, Amino, $C_1$-$C_4$-Alkylamino, Phenylamino oder einen Rest der Formel

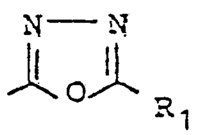

oder

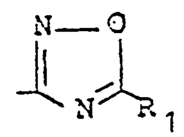

W Wasserstoff, gegebenenfalls durch Chlor oder cyano substituiertes $C_1$-$C_6$-Alkyl, Benzyl, Cyclohexyl oder Phenyl und

m eine ganze Zahl von 0 bis 7 bedeuten.

2. Verbindungen gemäß Anspruch 1, dadurch gekennzeichnet, daß

A für den Rest der Formel

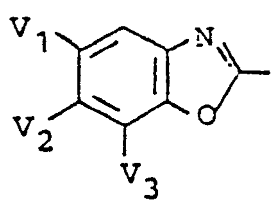

und

B für den Rest der Formel stehen

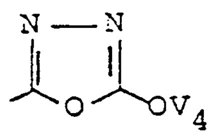

wobei n = 1 und worin

$V_1$ Wasserstoff, $C_1$-$C_4$-Alkyl, Cyclohexyl, $C_1$-$C_4$-Alkoxy, Chlor, Benzyl, Phenyl, $C_1$-$C_4$-Alkoxy-carbonyl, $C_1$-$C_4$-Alkylsulfonyl

$V_2$ Wasserstoff, $C_1$-$C_4$-Alkyl, Chlor oder $C_1$-$C_4$-Alkoxy

$V_3$ Wasserstoff, $C_1$-$C_4$-Alkyl oder Chlor und

$V_4$ gegebenenfalls durch $C_1$-$C_4$-Alkoxy, Hydroxy, Chlor oder Cyano substituiertes $C_1$-$C_6$-Alkyl, Benzyl, Cyclohexyl oder Phenyl bedeuten.

3. Verbindung gemäß Anspruch 1 der Formel

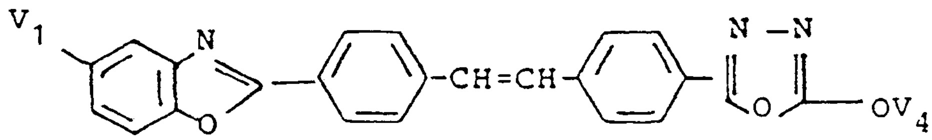

worin

$V_1$ für Wasserstoff, Methyl oder Chlor und

$V_4$ für Methyl, Ethyl, n-Propyl, Isopropyl oder n-Butyl stehen.

4. Verbindung der Formel

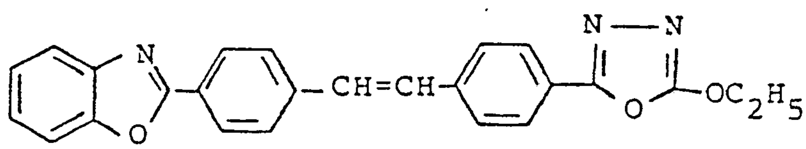

5. Verbindung der Formel

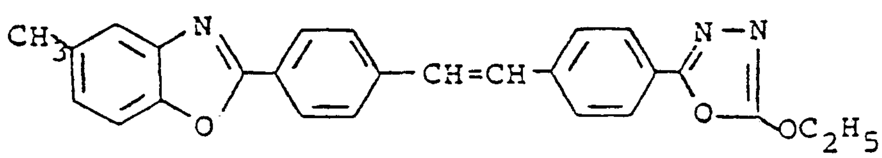

6. Verbindung der Formel

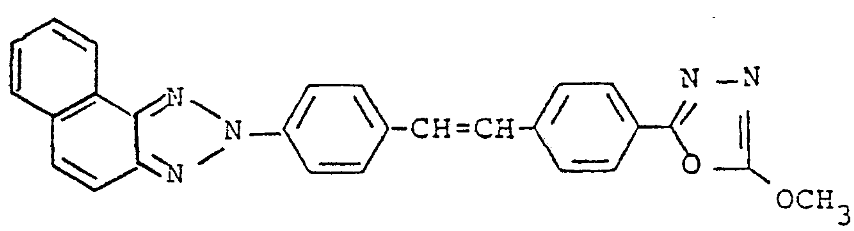

7. Verwendung der Verbindungen gemäß Anspruch 1 als optische Aufheller oder als Laserfarbstoffe.

**Claims**

1. Compounds of the formula

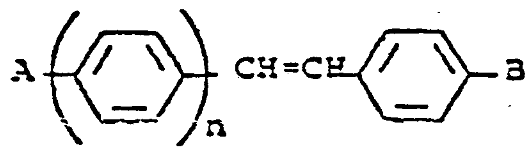

(I)

or mixtures of their isomers, wherein

A denotes a benzo-s-triazol-2-yl or benzoxazol-2-yl radical optionally substituted by R, R', OH, CN, OR, COR $SO_2R$ NHCOR, $CONH_2$, $NHSO_2R$, OCOR, COOR, COOH, NHR' or $SO_3H$, or a naphtho-s-triazol-2-yl radical, wherein R' represents alkyl and R represents alkenyl, aralkyl, cycloalkyl or aryl, B denotes a radical of the formula

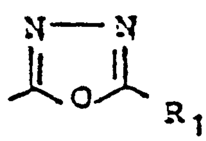

or

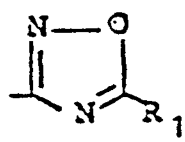

n denotes 1 or 2,

$R_1$ denotes hydrogen, chlorine, amino, $C_1$-$C_4$-alkylsmino, phenylamino or a radical of the formula

$$-(OCH_2CH_2)_m-OW$$

W denotes hydrogen, optionally chlorine- or cyanosubstituted $C_1$-$C_6$-alkyl, benzyl, cyclohexyl or phenyl and m denotes an integer from 0 to 7.

2. Compounds according to Claim 1, characterised in that A represents the radical of the formula

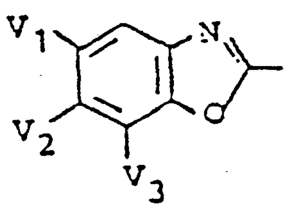

and B represents the radical of the formula

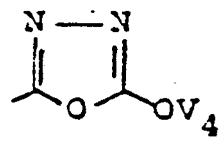

wherein .

n = 1 and wherein

$V_1$ denotes hydrogen, $C_1$-$C_4$-alkyl, cyclohexyl, $C_1$-$C_4$-alkoxy, chlorine, benzyl,; phenyl, $C_1$-$C_4$-alkoxycarbonyl or $C_1$-$C_4$-alkylsulphonyl,

$V_2$ denotes hydrogen, $C_1$-$C_4$-alkyl, chlorine or $C_1$-$C_4$-alkoxy,

$V_3$ denotes hydrogen, $C_1$-$C_4$-alkyl or chlorine and

$V_4$ denotes optionally $C_1$-$C_4$-alkoxy-, hydroxyl-, chlorineor cyano-substituted $C_1$-$C_6$-alkyl, benzyl, cyclohexyl or phenyl.

3. Compound according to Claim 1 of the formula

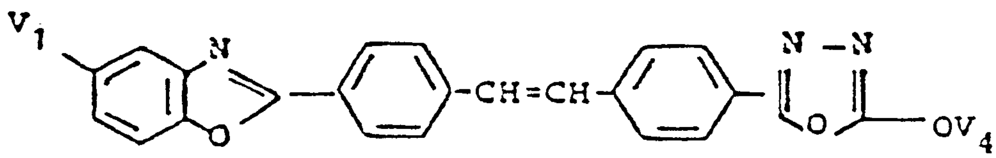

wherein

$V_1$ represents hydrogen, methyl or chlorine and

$V_4$ represents methyl, ethyl, n-propyl, isopropyl or n-butyl.

4. Compound of the formula

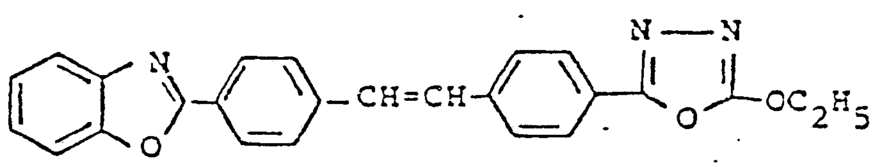

5. Compound of the formula

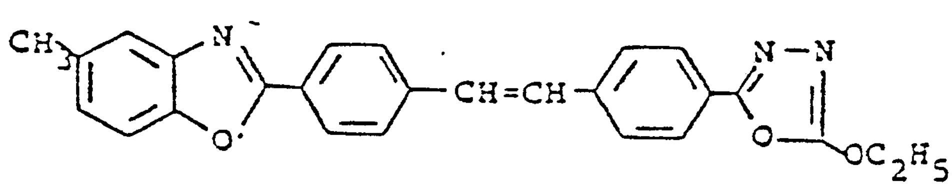

6. Compound of the formula

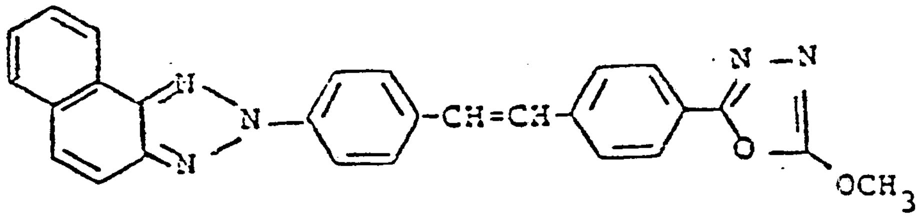

7. Use of the compounds according to Claim 1 as optical brighteners or as laser dyes.

**Revendications**

1. Composés de formule

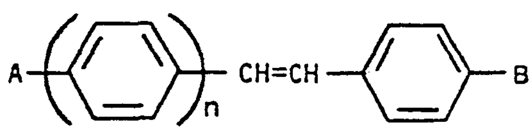

(I)

ou leurs mélanges d'isomères, dans lesquels

A représente un reste benzoxazolyle-2 ou benzo-s-triazolyle-2 éventuellement substitué par R, R', OH $CONH_2$, $NHSO_2R$, OCOR, COOR, COOH, NHR' ou $SO_3H$, ou un reste naphto s-triazolyle-2, R' représente un groupe alkyle et R un groupe alcényle, aralkyle, cycloalkyle ou aryle,

B représente un reste de formule

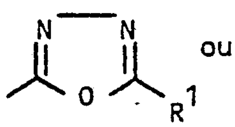

ou

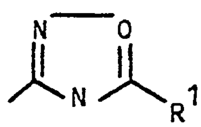

n est égal à 1 ou 2,

$R^1$ représente l'hydrogène, le chlore, un groupe amino, alkylamino en $C_1$-$C_4$

$$-(OCH_2CH_2)_m-OW$$

W représente l'hydrogène, un groupe alkyle en $C_1$-$C_6$, benzyle, cyclohexyle ou phényle, éventuellement substitué par le chlore ou des groupes cyano et

m est un nombre entier de 0 à 7.

2. Composés selon la revendication 1, caractérisés en ce que

A représente le reste de formule

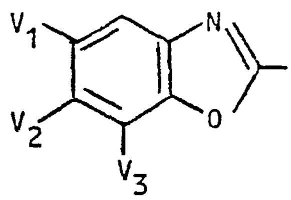

et

B représente le reste de formule

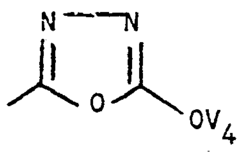

n égale 1 et

$V_1$ représente l'hydrogène, un groupe alkyle en $C_1$ -$C_4$ cyclohexyle, alcoxy en $C_1$-$C_4$, le chlore, un groupe benzyle, phényle, (alcoxy en $C_1$-$C_4$)-carbonyle, alkylsulfonyle en $C_1$-$C_4$,

$V_2$ représente l'hydrogène, un groupe alkyle en $C_1$-$C_4$,le chlore ou un groupe alcoxy en $C_1$-$C_4$,

$V_3$ représente l'hydrogène, un groupe alkyle en $C_1$-$C_4$ ou le chlore et

$V_4$ représente un groupe alkyle en $C_1$-$C_6$, benzyle, cyclohexyle ou phényle éventuellement substitué par des groupes alcoxy en $C_1$ -$C_4$, hydroxy, le chlore ou des groupes cyano.

3. Composé selon la revendication 1 de formule

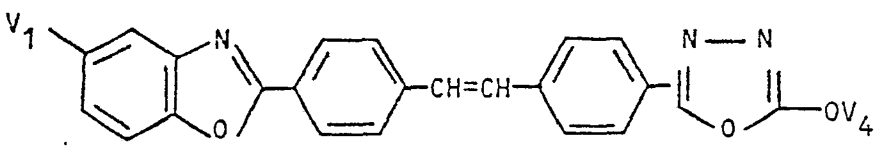

dans laquelle

$V_1$ représente l'hydrogène, un groupe méthyle ou le chlore et

$V_4$ représente un groupe méthyle, éthyle, n-propyle, isopropyl ou n-butyle.

4. Composé de formule

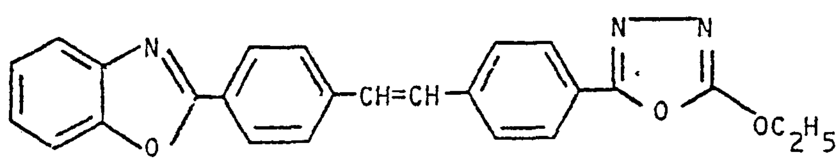

5. Composé de formule

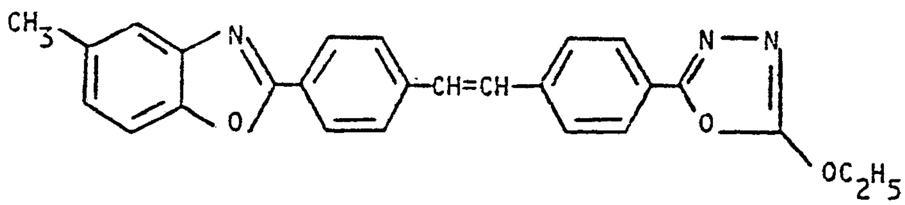

6. Composé de formule

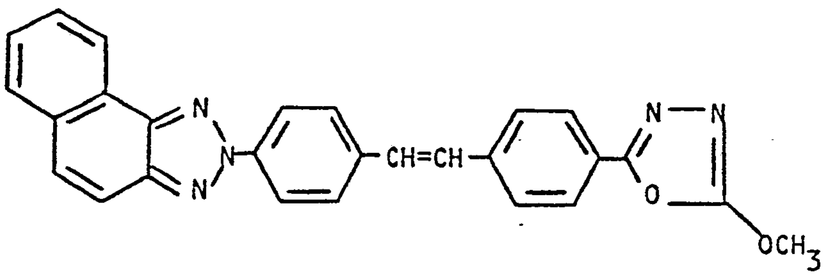

7. Utilisation des composés selon la revendication 1 en tant qu'azureurs optiques ou en tant que colorants lasers.